# EUROPÄISCHE PATENTANMELDUNG

(11) **EP 1 029 509 A1**
(43) Veröffentlichungstag der Anmeldung: **23.08.2000**
(21) Anmeldenummer: 99103264.0
(22) Anmeldetag: 19.02.1999
(51) Int. Cl.: A61B 17/32, A61M 1/00

(54) **Vorrichtung zum Schneiden und/oder Absaugen von Gewebeteilen oder dgl.**

(71) Anmelder: Magnetic Vision GmbH, 8630 Rüti (CH)
(72) Erfinder: Bernays, René, CH-8032 Zürich (CH); Viganò, Adriano, CH-8630 Rüti (CH)
(74) Vertreter: Troesch Scheidegger Werner AG

(57) **Zusammenfassung**

Die Erfindung betrifft eine Vorrichtung zum Schneiden und/oder Absaugen von Gewebeteilen oder dgl., wobei ein Rohr (2) vorgesehen ist, das Öffnungen im Bereich der Rohrenden aufweist. Erfindungsgemäss weist das Rohr (2) an einem Rohrende eine Spitze (2) auf, wobei mindestens eine Öffnung (F) im Bereich der Spitze (1) vorgesehen ist.

## Beschreibung

Die vorliegende Erfindung betrifft eine Vorrichtung nach dem Oberbegriff des Patentanspruchs 1.

In der Medizin werden verschiedene Instrumente und Methoden für die Entnahme von Gewebeteilen aus dem Innern des Körpers verwendet. So werden in der Neurochirurgie insbesondere Hämatome entfernt, indem der Schädel grossflächig, d.h. auf einer Fläche von mehreren Quadratzentimetern eröffnet wird. Tiefliegende Hämatome, d.h. etwa Hämatome im Bereich der Basalganglien, werden unter Verwendung herkömmlicher Operationstechniken nicht mehr operiert, da die Folgen der Verletzungen von wichtigem Nervengewebe die Vorteile einer Operation in der Regel überwiegen.

Bei der herkömmlichen Operationstechnik wird vor dem Eingriff ein Bild mittels Computertomographie erstellt. Nach dem Eröffnen der Schädeldecke mittels Operationspinzetten aus Stahl wird ein Zugang zur Läsion geschaffen. Dabei werden Nervenstrukturen und Blutgefässe teilweise verletzt. Nach Erreichen des Hämatoms wird dieses stückweise mittels Pinzetten und Saugern entfernt. Bei Anwendung der herkömmlichen Technik kann während der Operation die Vollständigkeit der Entfernung des Hämatoms nicht zuverlässig beurteilt werden, da eine interoperative Bildgebung fehlt bzw. nicht möglich ist.

Des weiteren ist eine Operationstechnik bekannt, die auf der Anwendung einer von Backlund im Jahre 1978 vorgeschlagenen stereotaktischen Methode beruht. Hierzu wird auf die druckschriftliche Veröffentlichung "Surgical Neurology" (1978, Seiten 99 bis 101) verwiesen. Die bekannte Lehre besteht darin, dass gerade und vorne offene Röhrchen zum Hämatom geführt werden, um dort die zu entfernenden Gewebeteile abzusaugen. Dabei kommt es allerdings häufig zu Obstruktionen, d.h. das Röhrchen wird durch abgetrennte Gewebeteile verstopft, weshalb diese Technik ohne Erfolg blieb.

Der vorliegenden Erfindung liegt daher die Aufgabe zugrunde, eine Vorrichtung anzugeben, die obenerwähnte Nachteile nicht aufweist.

Diese Aufgabe wird durch die im kennzeichnenden Teil des Patentanspruchs 1 angegebenen Massnahmen gelöst. Vorteilhafte Ausgestaltungen der Erfindung sind in weiteren Ansprüchen angegeben.

Die Erfindung weist die folgenden Vorteile auf: Indem ein Rohr eine seitliche Öffnung und eine Spitze im Bereich der Öffnung aufweist, wird bei minimaler Gewebeverletzung von gesundem Gewebe, d.h. beispielsweise des Gehirns, eine effektive Entfernung von kranken Gewebeteilen, d.h. beispielsweise ein Hämatom, erreicht wird.

Mit der erfindungsgemässen Vorrichtung wird in einer ersten Verwendungsphase die Spitze des Rohres ins Zentrum des zu entfernenden Gewebeteils vorgeschoben. Daraufhin werden durch das zugeschaltete Vakuum direkt vor der Öffnung liegende Gewebeteile angesaugt. Durch ein axiales Drehen des Rohres werden die angesaugten Gewebeteile abgetrennt und können so quasi portionsweise abgesaugt werden, ohne dass es zu einer Obstruktion im Rohr kommen kann. Dieser Prozess ist repetitiv und wird solange durchgeführt, bis die gewünschten Gewebeteile entfernt sind.

Die Spitze des Rohres ist dabei derart ausgebildet, dass Hirngewebe und Blutgefässe beim Vorschieben des Rohres zur Seite gedrängt, also weder gequetscht noch durchgetrennt werden.

Der Durchmesser des Rohres ist so bemessen, dass eine minimale Traumatisierung und eine maximale Evakuationsleistung erhalten wird. Die erfindungsgemässe Vorrichtung eignet sich vorzüglich in Verbindung mit dem Snapper-Stereoguide, der in der internationalen Patentanmeldung mit der Veröffentlichungsnummer WO 98/17191 beschrieben ist.

Die seitlich angebrachte Öffnung im Bereich der Spitze des Rohres ist so beschaffen, dass genügend grosse Gewebsstücke angesaugt und durch axiale Drehung des Rohres abgetrennt werden können.

Als Material für erfindungsgemässe Vorrichtung eignen sich insbesondere nicht metallische Stoffe, d.h. besonders Polymere, die nebst einer artefaktfreien Abbildung der Vorrichtung zum Beispiel mittels MRI (Magnetic Resonance Imaging) oder CT (Computer Tomography) auch eine weitgehende Schonung vitaler Strukturen, insbesondere der Blutgefässe erlauben.

Das Innenrohr, das sich beim Einführen der erfindungsgemässen Vorrichtung innerhalb des Rohres befindet, verhindert zusätzlich eine mögliche Verletzung von Hirngewebe im Bereich der schneidenden Öffnung.

Die Dosiereinheit gibt nur dann ein Vakuum an die Öffnung weiter, wenn mittels Finger des Operateurs das schlitzförmige Fenster teilweise oder ganz verschlossen wird. Mittels manueller gradueller Schliessung des Dosierfensters wird das Evakuationsvakuum reguliert.

Das an die Dosiereinheit anschliessende, abgewinkelte Durchsichtsfenster ermöglicht eine zusätzliche visuelle Kontrolle des abgesaugten Materials durch den Operateur.

Die Erfindung wird nachfolgend anhand von Zeichnungen beispielsweise näher erläutert. Dabei zeigen
- Fig. 1: eine erfindungsgemässe Vorrichtung zum Schneiden und/oder Absaugen von Gewebeteilen,
- Fig. 2: die Vorrichtung gemäss Fig. 1 in teilweiser explosionsartiger Darstellung,
- Fig. 3: eine mit der Vorrichtung gemäss Fig. 1 und 2 verbindbare Dosiereinheit,
- Fig. 4: die Dosiereinheit gemäss Fig. 3 in explosionsartiger Darstellung,
- Fig. 5: ein zur Vorrichtung gemäss Fig. 1 und 2 gehörendes Rohr in Detailansicht,
- Fig. 6: eine Ansicht der Öffnung im Rohr zum Schneiden von Gewebeteilen und
- Fig. 7: einen Schnitt A-A gemäss Fig. 5 durch das Rohr im Bereich der Öffnung.

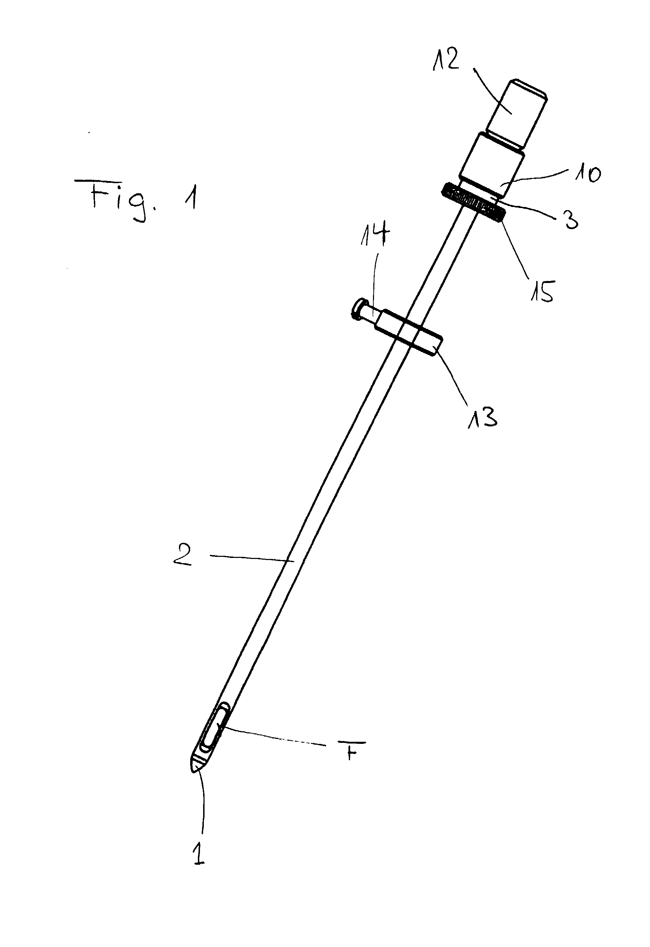

Fig. 1 zeigt, in perspektivischer Darstellung, eine erfindungsgemässe Vorrichtung, bestehend aus einem Rohr 2, das am einen Ende eine Spitze 1 und am anderen Ende eine Öffnung (in Fig. 1 nicht sichtbar) aufweist. Des weiteren ist im Rohr 2 eine weitere Öffnung F im Bereich der Spitze 1, vorzugsweise in geringem Abstand zu dieser vorgesehen. Diese weitere Öffnung F wird anhand der Figuren 5 bis 7 ausführlich erläutert.

Am Rohr 2 ist ferner ein Handrad 15 fixiert, das zum einfachen und präzisen Führen durch den behandelnden Arzt einerseits einen wesentlich grösseren Radius als das Rohr 2 und anderseits eine griffige Oberfläche aufweist. Mit 10 ist eine Überwurfmutter bezeichnet, die mit dem Rohr 2 bzw. mit einer in Fig. 1 nur eingeschränkt sichtbaren Verbindungshülse 3 verbindbar ist.

Des weiteren ist ein Anschlag 13 vorgesehen, der mit einem Befestigungselement 14, vorzugsweise einer Schraube oder einer Federklemme, lösbar mit dem Rohr 2 verbunden werden kann.

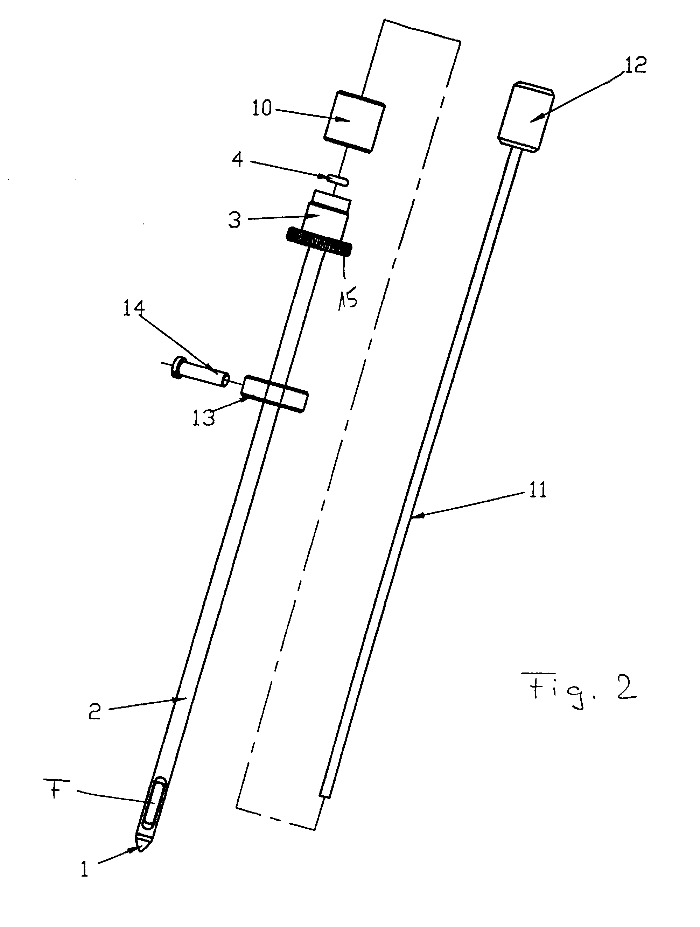

Schliesslich ist mit 12 eine Abschlusshalterung bezeichnet, die mit einem anhand Fig. 2 zu erläuternden Innenrohr (in Fig. 1 nicht ersichtlich) verbunden ist.

Fig. 2 zeigt die in Fig. 1 dargestellte Vorrichtung in teilweiser explosionsartiger Darstellung. Dabei ist nunmehr die mit einem Innenrohr 11 verbundene Abschlusshalterung 12 ersichtlich, deren Aussenradius deutlich grösser ist als der Aussenradius des Innenrohres 11, womit dessen Lage zu jeder Zeit kontrollierbar ist. Die Abmessungen des Innenrohrs 11 sind so gewählt, dass bei vollständiger Aufnahme im Rohrmantel 2 die Öffnung F vollständig geschlossen ist.

In Fig. 2 ist die erwähnte Verbindungshülse 3 vollständig erkennbar, in die ein Dichtungsring 4, vorzugsweise in Form eines O-Ringes, einsetzbar ist, wofür eine entsprechende Ausnehmung (in Fig. 2 nicht dargestellt) in der Verbindungshülse 3 vorgesehen ist. Die Verbindungshülse 3 weist auf deren Aussenseite ein Gewinde auf, das in ein entsprechendes Gewinde auf der Innenseite der Überwurfmutter 10 eingreift.

Die Überwurfmutter 3, die Verbindungshülse 3 und der bei einer bevorzugten Ausführungsform der Erfindung vorgesehene Dichtungsring 4 sind Mittel zum lösbaren Anschliessen einer Leitung zu einer Vakuumpumpe, was nachstehend weiter erläutert wird.

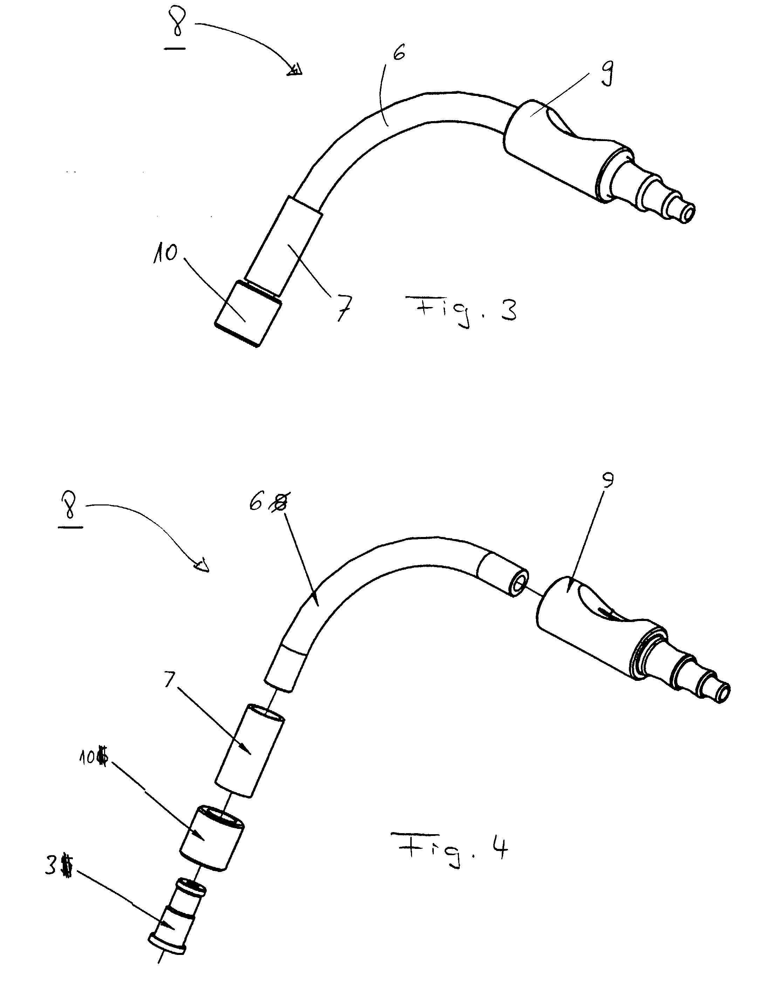

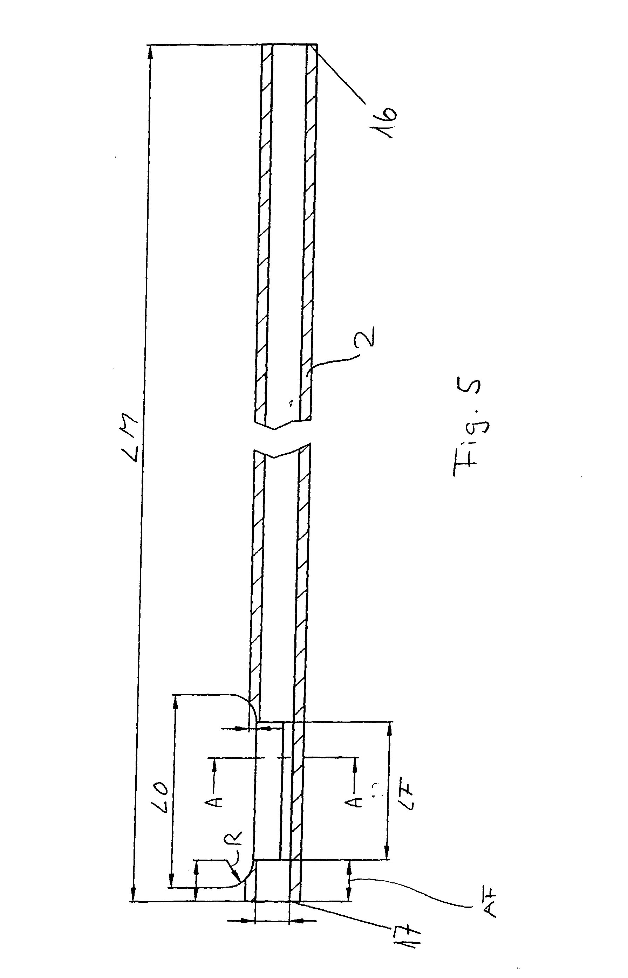

In den Fig. 3 und 4 ist eine bevorzugte Ausführungsform eines Teilstücks der an das Rohr 2 (Fig. 1 und 2) anschliessbaren Leitung 8 dargestellt. Dieses Teilstück besteht aus der Überwurfmutter 10, einem Zylinderelement 7, einem mit 6 bezeichneten Abschnitt der Leitung 8, der vorzugsweise aus einem starren und transparenten Bogenrohr besteht, und einem Dosierelement 9, an das weitere Leitungsabschnitte, beispielsweise ein flexibler Schlauch, anschliessbar sind. Das Ende der Leitung 8 ist schliesslich mit der erwähnten Vakuumpumpe zu verbinden.

Das Dosierelement 9 ist zylinderförmig und weist einen Radius auf, der verglichen mit dem Radius des Bogenrohres 6 bzw. des Schlauches wesentlich grösser ist. Im mittleren Bereich der Zylinderoberfläche des Dosierelementes 9 ist eine Ausnehmung in Form einer Mulde vorgesehen, in der eine Öffnung in Form eines Schlitzes enthalten ist.

In Fig. 4 sind die einzelnen Bestandteile des anhand Fig. 3 erläuterten Leitungsabschnittes dargestellt, wobei zusätzlich die mit dem Rohr 2 (Fig. 1 und 2) verbundene Verbindungshüle 3 mit eingesetztem Dichtungsring abgebildet ist.

In Fig. 5 ist ein Längsschnitt durch das Rohr 2 (Fig. 1 und 2) mit einer Länge LM dargestellt, wobei die Schnittebene durch die Mittelachse des Rohres und durch die Symmetrieebene der Öffnung F geführt ist. Mit 16 ist die mit dem Verbindungselement 3 und mit 17 die mit der Spitze 1 zu verbindende Stirnseite des Rohres 2 gekennzeichnet. Im Bereich der Spitze 1 des Rohres 2, d.h. in einem verglichen mit der Rohrlänge LM geringen Abstand AF, weist das Rohr 2 eine Öffnung F mit der Länge LF auf, wobei der Übergang von der Öffnung F zur Rohroberfläche in Richtung der Längsachse des Rohres 2 flach, vorzugsweise entsprechend der Form eines Kugelsektors mit Radius R ausgebildet ist, so dass eine die Öffnung F enthaltende Ausnehmung entsteht, die sich über die parallel zur Rohrachse verlaufende Länge LO erstreckt.

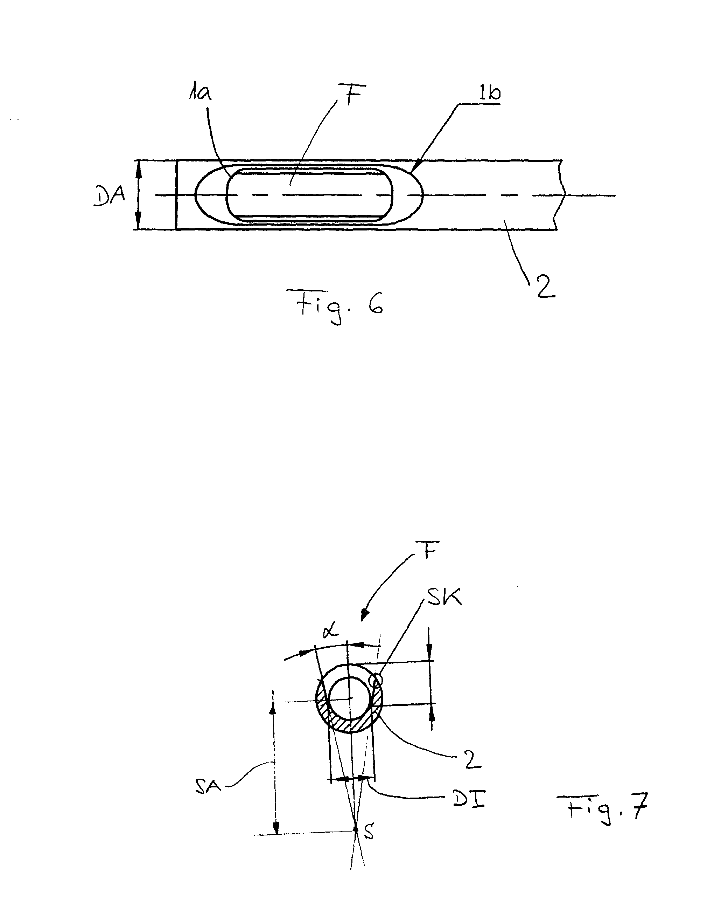

Fig. 6 zeigt eine Ansicht der Öffnung 7 im Rohr 2 und verdeutlicht den anhand von Fig. 5 erläuterten Aufbau weiter. Dabei ist mit 1b die äusseren Konturen der Ausnehmung im Rohr 2 und mit 1a die eigentliche Öffnung F bezeichnet.

Schliesslich ist in Fig. 7 ein Schnitt durch die in Fig. 5 definierte Ebene A-A dargestellt. Die Öffnung F ist durch einen Kreissektor definiert, der einen Scheitelpunkt S und einen Öffnungswinkel α aufweist, wobei der Scheitelpunkt S in einem Abstand SA zur Mittelachse des Rohres 2 liegt. Das Verhältnis von Abstand SA und Innendurchmesser DI liegt vorzugsweise zwischen 3 und 4. Dadurch wird eine für den noch zu erläuternden Schneidevorgang benötigte optimale Kante SK erhalten.

Im folgenden wird die Verwendung der erfindungsgemässen Vorrichtung als Hämatomevaquator erläutert:

Die in Fig. 1 dargestellt Vorrichtung wird durch eine Führungshülse eines sogenannten "Snapper Stereoguide" - wie er beispielsweise in der Internationalen Patentanmeldung WO 98/17191 beschrieben ist - in den Schädel eines Patienten bis zu einer gewünschten Stelle eingeführt. Dabei ist von entscheidender Bedeutung, dass das Innenrohr 11 im Rohr 2 zur Verschliessung der Öffnung F enthalten ist, damit möglichst wenig Gehirnmasse verletzt wird. Die Form der Spitze 1 ist dabei derart ausgelegt, dass beim Einführen der erfindungsgemässen Vorrichtung das Gewebe zur Seite gedrängt und nicht durchtrennt wird.

Ist die Eindringtiefe vor dem Eingriff bestimmt worden, so kann diese mittels dem Anschlag 13 bzw. den diesen fixierenden Befestigungselementen 14 entsprechend eingestellt werden. Andernfalls kann, unter Verwendung von entsprechenden bildgebenden Instrumenten - die Eindringtiefe laufende überwacht werden, wobei dann der Anschlag 13 bei Erreichen der gewünschten Position der Öffnung F fixiert wird, womit ein weiteres Vordringen der Spitze 1 verhindert wird.

Das Innenrohr 11 wird nun durch Ziehen an der Abschlusshalterung 12 aus dem Rohr 2 herausgezogen. Anschliessend wird die Überwurfmutter 10 abgeschraubt und die in Fig. 3 dargestellte Dosiereinheit an das Rohr 2 bzw. dessen Verbindungshülse 3 angeschlossen. Ein mit der Vakuumpumpe verbundener Schlauch wird an das Dosierelement 9 angeschlossen, wodurch Luft über den Schlitz in der Mulde des Dosierelementes 9 gesaugt wird.

Mit dem Daumen kann der Chirurg die Öffnungsweite des Schlitzes variieren und somit das Vakuum kontrolliert an die Öffnung F im Rohr 2 weiterleiten. Während eines solchen Saugvorganges durch die Öffnung F wird das Rohr 2 durch Betätigen des Handrades 15 gedreht, wobei durch die drehbare Lagerung der Verbindungshülse 3 gegenüber dem Dichtungselement 4 und der Überwurfmutter 10 die Dosiereinheit 9 an Ort und Stelle verbleiben kann, was eine einfache Handhabung ermöglicht. Die durch die Öffnung F in das Innere des Rohres 2 gesaugten Gewebeteile werden infolge der Rotation des Rohres 2 durch die Kanten SK abgeschnitten und über das Rohr 2, den Rohrbogen 8 und den Schlauch in Richtung Vakuumpumpe transportiert. Der Transport von abgeschnittenen Gewebeteilen kann bei Verwendung eines transparenten Bogenrohres 6 visuell überwacht werden.

In einer bevorzugten Ausführungsform sind sämtliche Bestandteile der erfindungsgemässen Vorrichtung aus einem nicht metallischen Material bzw. aus Teflon gefertigt.

Die erfindungsgemässe Vorrichtung eignet sich nicht nur vorzüglich als Hämatomevaquator sondern insbesondere auch für sämtliche Gewebeentnahmen am menschlichen oder tierischen Körper.

## Patentansprüche

1. Vorrichtung zum Schneiden und/oder Absaugen von Gewebeteilen oder dgl., wobei ein Rohr (2) vorgesehen ist, das Öffnungen (F) im Bereich der Rohrenden aufweist, dadurch gekennzeichnet, dass das Rohr (2) an einem Rohrende eine Spitze (1) aufweist und dass mindestens eine Öffnung (F) im Rohrmantel im Bereich der Spitze (1) vorgesehen ist.

2. Vorrichtung nach Anspruch 1, dadurch gekennzeichnet, dass die Öffnung (F) derart ausgebildet ist, dass beim Drehen des Rohres (2) ein in die Öffnung (F) ragendes Gewebeteil abgeschnitten wird.

3. Vorrichtung nach Anspruch 1 oder 2, dadurch gekennzeichnet, dass in das Rohr (2) ein Innenrohr (11) aufnehmbar ist, mit Hilfe dessen die Öffnung (F) verschliessbar ist.

4. Vorrichtung nach einem der vorangehenden Ansprüche, dadurch gekennzeichnet, dass am Rohr (2) ein Anschlag (13) lösbar fixiert ist.

5. Vorrichtung nach einem der vorangehenden Ansprüche, dadurch gekennzeichnet, dass das Rohr (2) ein Handrad (15) aufweist.

6. Vorrichtung nach einem der vorangehenden Ansprüche, dadurch gekennzeichnet, dass Mittel (3, 10) zum lösbaren Anschliessen einer Leitung (8) an das Rohr (2) vorgesehen sind, wobei die Mittel (3, 10) derart ausgestaltet sind, dass das Rohr (2) in bezug auf die Leitung (8) drehbar sind.

7. Vorrichtung nach Anspruch 6, dadurch gekennzeichnet, dass zumindest ein Abschnitt der Leitung (8) ein Bogenrohr (6) ist, das vorzugsweise unmittelbar an das Rohr (2) anschliessbar ist.

8. Vorrichtung nach Anspruch 7, dadurch gekennzeichnet, dass das Bogenrohr (6) transparent ist.

9. Vorrichtung nach Anspruch 7 oder 8, dadurch gekennzeichnet, dass die Leitung (8) an eine Vakuumpumpe anschliessbar ist, wobei zwischen Vakuumpumpe und Rohr (2) ein Dosierelement (9) vorgesehen ist.

10. Vorrichtung nach Anspruch 9, dadurch gekennzeichnet, dass das Dosierelement (9) aus einem zylinderförmigen Zwischenelement mit einer Ausnehmung in Form einer Mulde besteht, in der eine Öffnung in Form eines Schlitzes enthalten ist.

11. Vorrichtung nach einem der Ansprüche 6 bis 10, dadurch gekennzeichnet, dass die Mittel zum lösbaren Anschliessen der Leitung (8) aus einer mit dem Rohr (2) verbundenen Verbindungshülse (5), die auf deren Aussenseite ein Gewinde aufweist, aus einem in die Verbindungshülse (3) eingelassenen Dichtungselement (4) und aus einer Überwurfmutter (10), die über ein Zylinderelement (7) mit der Leitung (8) verbindbar ist, besteht.

12. Vorrichtung nach einem der vorangehenden Ansprüche, dadurch gekennzeichnet, dass alle Bestandteile der Vorrichtung aus einem nicht metallischen Material gefertigt sind.
